# EUROPEAN PATENT SPECIFICATION

(11) **EP 1 441 746 B1**
(45) Date of publication and mention of the grant of the patent: **24.12.2008**
(21) Application number: 02786665.6
(22) Date of filing: 06.11.2002
(51) Int. Cl.: A61K 33/04, A23K 1/175, A23K 1/18

(54) **COMPOSITION AND METHOD**
ZUSAMMENSETZUNG UND VERFAHREN
COMPOSITION ET METHODE

(30) Priority: 07.11.2001 US 683003
(43) Date of publication of application: 04.08.2004
(73) Proprietor: Hill's Pet Nutrition, Inc., Topeka, KS 66603 (US)
(72) Inventor: YU, Shiguang, Topeka, KS 66610 (US); NACHREINER, Raymond, East Lansing, MI 48823 (US); KIRK, Claudia, Ann, Lawrence, KS 66047 (US); WEDEKIND, Karen, J., Meriden, KS 66512 (US)
(74) Representative: Winckels, Johannes Hubertus F.
(86) International application number: PCT/US2002/035546
(87) International publication number: WO 2003/039562

(56) References cited:
- EP-A- 0 583 026
- GB-A- 1 444 024
- US-A- 5 629 002
- US-A- 6 156 355
- US-B1- 6 238 708
- LEE, J., MASTERS, D. G., WHITE, C. L., GRACE, N. D., AND JUDSON, G. J.: "Current issues in trace element nutrition of grazing livestock in Australia and New Zealand" AUSTRALIAN JOURNAL OF AGRICULTURAL RESEARCH, vol. 50, no. 8, 1999, pages 1341-1364, XP008013354 MELBOURNE, AU
- DATABASE WPI Section Ch, Week 199727 Derwent Publications Ltd., London, GB; Class D13, AN 1997-289978 XP002230564 & CN 1 103 558 A (MEAT PIGEON COMPREHENSIVE TECHNOLOGY DEV), 14 June 1995 (1995-06-14)

## Description

### BACKGRDUND OF THE INVENTION

Selenium is well known as an essential component of a mammal's diet. Many functions have been associated with it These include growth, reproduction, antioxidant, endocrine function, immune function, normal hair growth, muscle, and heart function. Selenium has been used in hair shampoos as an antidandruff agent, see USP 5,798, 121 and 5,702,690 as the disulphide and sulphide. Topical application of selenium has been implicated in hair growth, see USP 5,629,002 wherein inorganic selenium compounds together with thiocyanic compounds are disclosed as improving quality and stimulating the growth of hair. At column 1, lines 28-44 of 002, various materials including selenium, are disclosed as:
"can display a more or less stimulating influence on the growth of
the hair. However, the affects achieved thereby, as a rule, are not
significant and not reproducible in practise."

Example 9 of US-A-5,629,002 relates to a composition comprising 0.1 mg of a selenium salt in water. The selenium content is thereby higher than 2.0 mg/kg on dry matter.

US 6,238,708 B! discloses a composition and process for controlling postprandial glycemic and/or insulinemic response in companion animals such as dogs. Table 8 shows a composition comprising 0.27 mg selenium per kg dry matter, presumably in the mineral mix. It may comprise 0.4 mg/kg selenium. Maintaining a healthy skin and hair coat is associated with ω-6 and ω-3 polyunsaturated fatty acids; not with selenium

The review article in the Australian Journal of Agricultural Research (1999), 50(8), 1341-1364, to J. Lee, D.G. Masters, C.J. White, N.D. Grace and G.J. Judson (Current issues in trace element nutrition of razing livestock in Australia and New Zealand) refers to improving trace element nutrition of grazing animals, in a way that is cost effective and that meets consumer perceptions and preferences. There is no reference to a dog or cat diet composition.

CN 1103558 discloses an oral nutrient product which composition contains 0.5-350 ppm Se. The composition is said to regulate physiological function of body while making up nutrient and is especially suitable for children lacking zinc, postpartum women and postoperative patients. It is stated that the composition has the health functions of removing freckles, preventing alopecia, preventing senility, etc. The composition is said to be suitable for a.o. treating alopecia, though there is no pointer to what the effective compound is.

We have now found a range of dietary selenium that stimulates hair growth of dogs and cats receiving a properly nutritious diet. Quantitative measures show hair growth rate is statistically greater within this range. Below and above the range, hair growth is slowed. Providing a steady positive growth rate for hair can be desirable for esthetic benefits. Enhancing hair growth with selenium supplementation can benefit the management of disorders of the hair follicles and/or hair coat by preventing or treating poor hair growth or alopecia related to disease, reproductive cycle, seasonal changes or direct hair removal (e.g., shaving or clipping).

### SUMMARY OF THE INVENTION

In accordance with the invention, there is a dog or cat diet composition which comprises from 0.5 to 2.0 mg selenium/kg of diet on a dry matter basis, the diet providing nutritional sustenance. Generally, hair growth is slowed when dietary selenium concentration is below 0.1 mg selenium/kg diet and above 4.5 mg selenium/kg diet on a dry matter basis. Thus, the range between 0.5 and 2.0 mg selenium/kg diet maximizes hair growth.

A further aspect of the invention is the use of a composition according to the invention in the manufacture of a dog or cat diet for controlling the rate of hair growth in a dog or cat the diet providing nutritional sustenance. Otherwise, the selenium can be fed the dog or cat as a supplement to the regular diet as long as the appropriate levels of selenium are maintained.

### DETAILED DESCRIPTION OF THE INVENTION

Selenium can be administered orally to the dog or cat in any form as long as it is absorbed by the animals. Examples of such forms of selenium include salts such as selenites and selenates, for example, sodium selenite, and sodium selenate; acids such as selenious and selenic acid; organic compounds containing selenium such as selenomethionine, selenocysteine, selenohomocystine, selenotaurine; and selenium containing food or ingredients such as meat, fish, and the like.

The quantity of selenium as disclosed in this specification is measured as the quantity of selenium per se. For example, if selenium is administered as selenomethionine, it is the actual quantity of selenium that is in the selenomethionine that is intended as the quantity disclosed, The minimum quantity of selenium that brings about the controlled rate of hair growth in cats and dogs is 0.1 mg as measured per kg diet on dry matter basis. The maximum quantity is a controlling hair growth rate but non-toxic quantity of selenium. Generally, this is no more than 4.5 mg, desirably no more than 2 mg per kg diet on dry matter basis. A desirable minimum is 0.5 mg per kg diet on dry matter basis. As stated previously, it has now been found that the control of hair growth rate through usage of selenium can result in a pronounced statistically significant hair growth acceleration depending upon the quantity of selenium employed. Hair growth rate increases and reaches its maximum when dietary selenium concentration is above 0.1 mg selenium/kg diet and hair growth is slowed when dietary selenium concentration is higher than 4.5 mg selenium/kg. Selenium can be administered to the dog or cat in diet or through specific supplements to be fed to the animal.

Animals that benefit from increased hair growth rate are dogs and cats.

In addition to optimizing growth of hair coat for esthetic benefit, enhancing hair growth with selenium supplementation can benefit the management of disorders of the skin, hair follicle and/or hair coat by preventing or treating poor hair growth or alopecia related to disease, reproductive cycle, seasonal changes or direct hair removal (e.g., shaving or clipping).

Below is an example of the invention showing the benefit and effect of using selenium.

### Example 1

Adult beagles of both male and females are used. They are between 1.2 and 3.7 years of age at the start of the experiment

Study Design: Thirty-six adult dogs are divided into six groups of six dogs in each group based on their age, gender and body condition score so that the averages of those three criteria are similar among groups. All dogs are given the basal diet for three weeks (pre-treatment period) before they are allocated to one of the experimental diets. The third week of the pre-treatment period is considered as week 0 of the study. Hair growth is measured in weeks 0,11 and 22 of the study using the method described below. Dogs are fed with the experimental diets for 24 weeks.

A torula yeast basal diet is used. The basal diet is nutritionally complete and balanced for an adult dog except for selenium. Different amounts of selenium (as selenomethionine) are added into the basal diet to form six experimental diets which contain 0, 0.05, 0.1, 0.5, 1 or 5 mg selenium/kg diet dry matter, respectively. Selenium concentrations in the experimental diets are confined by chemical analyses- The analyzed dietary selenium concentrations were 0.034, 0.085, 0.123, 0.527, 1.025 and 5.045 mg/kg dry matter, respectively.

Hair Growth Measurement: Hairs in an area of 2" x 2" on the left side of the body above the hind leg and about 3 cm away from the spinal are clipped with an electronic clipper (Oster Pro™ Model 78400-01A, blade size: 40.1mm, Oster Elite™). The hair is cut as close to the skin as possible. A tattoo dot of about 1 mm in diameter is made in the center of the clipped area using a tattoo marker (Spaulding Special Electric Tattoo Marker, Model SSEMK). Hair growth is measured by taking photographs from the same clipped area twice using the tattoo dot as a marker. The first photograph is taken right after the clipping and the second photograph four days later. A Polaroid close-up camera (CU-5) and Polaroid 665 films are used to take photographs. A glass slide is attached to the front frame of the camera to press hairs to the skin to minimize errors caused by the angle formed between standing hair and the skin. The image is magnified three times by the camera. Same exposure conditions are used for all dogs in the study. On each day of photograph shooting, a photograph is also taken of a ruler with clear markers under the same conditions and serves as a reference. After exposure, both prints and negatives of the films are treated according to the use directions. The negative films provide sharp and clear images of hairs and are used for hair growth measurement. The negative films are scanned (EPSON Expression 836XL) into a computer. Afterwards, the length of primary hairs in the film is measured using an image analyzing software (BioScan Optimas). The difference of the average hair lengths between the first and the second photographs from the same dog is the hair growth during the four-day period.

Results: There is no difference in hair growth among groups in week 0 (Table 1). However, dogs given diets containing 0.1, 0.5 or 1 mg selenium/kg diet have a significantly (p<0.05) higher rate of hair growth than dogs receiving diets with 0, 0.05 or 5 mg selenium/kg diet in week 11 and 22 of the study.

**Table 1**

| Hair growth of Beagles fed diets with various levels of selenium | | | |
|---|---|---|---|
| | Hair growth (mm/day) | | |
| Dietary Se (mg/kg) | Week 0 | Week 11 | Week 22 |
| 0.034 | 0.21 | 0.12 | 0.12 |
| 0.085 | 0.18 | 0.15 | 0.11 |
| 0.123 | 0.13 | 026 | 0.20 |
| 0.527 | 0.15 | 0.22 | 0.23 |
| 1.025 | 0.18 | 0.26 | 0.20 |
| 5.045 | 0.20 | 0.14 | 0.13 |

No apparent clinical signs of selenium deficiency or toxicity are observed in the dogs during the study period. Average food intake and body weights are not affected by the dietary treatments. Blood CBC and chemistry measurements as well as serum total thyroxine and 3, 5, 3' - triiodothyronine are within the normal ranges. These results demonstrate that dietary selenium concentrations from 0.1 to 4.5 mg selenium/kg diet maximize hair growth of dogs, with preferred levels between 0.1 and 2 mg selenium/kg diet and most preferred at 0.1 to 1 mg selenium/kg diet dry matter.

## Claims

1. A dog or cat diet composition which comprises from 0.5 to 2.0 mg selenium per kg of diet on a dry matter basis.

2. Use of the composition of claim 1 in the manufacture of a dog or cat diet for controlling the rate of hair growth in a dog or cat.

3. Use of the composition of claim 1 in the manufacture of a dog or cat diet for preventing or treating poor hair growth or alopecia in dogs or cats in need of said treatment, related to disease, reproduction cycle, seasonal changes or direct hair removal.

## Patentansprüche

1. Nahrungsmittelzusammensetzung für Hunde oder Katzen, die von 0,5 bis 2,0 mg Selenium pro kg des Nahrungsmittels im trockenen Zustand enthält.

2. Verwendung der Zusammensetzung nach Anspruch 1 für die Herstellung eines Nahrungsmittels für Hunde oder Katzen zur Regulierung des Haarwuchses bei Hunden oder Katzen.

3. Verwendung der Zusammensetzung nach Anspruch 1 für die Herstellung eines Nahrungsmittels für Hunde oder Katzen zur Verhinderung oder Behandlung von durch Krankheit, den Fortpflanzungzyklus, jahreszeitlichen Schwankungen oder direkter Haarentfernung verursachten schlechtem Haarwuchs oder Haarausfall bei Hunden oder Katzen, die einer solchen Behandlung bedürfen.

## Revendications

1. Composition de nourriture pour chien ou chat qui comprend de 0,5 à 2,0 mg de sélénium par kg de nourriture sur la base de la matière sèche.

2. Utilisation de la composition selon la revendication 1 pour la fabrication d'une nourriture pour chien ou chat pour contrôler la vitesse de croissance des poils d'un chien ou d'un chat.

3. Utilisation de la composition selon la revendication 1 pour la fabrication d'une nourriture pour chien ou chat, pour prévenir ou traiter une alopécie ou une mauvaise croissance des poils liée à une maladie, au cycle de reproduction, à des changements saisonniers ou à la tonte directe des poils, chez des chiens ou des chats ayant besoin dudit traitement.
